# EUROPEAN PATENT SPECIFICATION

(11) **EP 2 757 941 B1**
(45) Date of publication and mention of the grant of the patent: **11.05.2022**
(21) Application number: 12833897.7
(22) Date of filing: 17.09.2012
(51) Int. Cl.: A61B 5/30, A61B 5/374, A61B 5/00

(54) **SYSTEM FOR ANALYZING AN EEG RECORDING**
SYSTEM ZUR ANALYSE EINER EEG-AUFZEICHNUNG
SYSTÈME D'ANALYSE D'UN ENREGISTREMENT EEG

(30) Priority: 19.09.2011 US 201161536236 P; 15.09.2012 US 201213620855
(43) Date of publication of application: 30.07.2014
(73) Proprietor: Persyst Development Corporation, Solana Beach, CA 92075 (US)
(72) Inventor: WILSON, Scott, B., San Diego, CA 92130 (US); NIERENBERG, Nicolas, San Diego, CA 92130 (US); SCHEUER, Mark, San Diego, CA 92130 (US)
(74) Representative: Tomkins & Co
(86) International application number: PCT/US2012/055692
(87) International publication number: WO 2013/043517

(56) References cited:
- EP-A1- 0 013 183
- WO-A1-2008/061292
- WO-A2-2008/038194
- WO-A2-2008/057365
- RU-C1- 2 415 642
- US-A1- 2005 059 874
- US-A1- 2007 161 919
- US-A1- 2009 247 895
- US-A1- 2011 015 537

## Description

### Technical Field

The present invention generally relates to EEG recordings. More specifically, the present invention relates to analyzing an EEG recording.

### Background Art

An electroencephalogram ("EEG") is a diagnostic tool that measures and records the electrical activity of a person's brain in order to evaluate cerebral functions. Multiple electrodes are attached to a person's head and connected to a machine by wires. The machine amplifies the signals and records the electrical activity of a person's brain. The electrical activity is produced by the summation of neural activity across a plurality of neurons. These neurons generate small electric voltage fields. The aggregate of these electric voltage fields create an electrical reading which electrodes on the person's head are able to detect and record. An EEG is a superposition of multiple simpler signals. In a normal adult, the amplitude of an EEG signal typically ranges from 1 micro-Volt to 100 micro-Volts, and the EEG signal is approximately 10 to 20 milli-Volts when measured with subdural electrodes. The monitoring of the amplitude and temporal dynamics of the electrical signals provides information about the underlying neural activity and medical conditions of the person.

An EEG is performed to: diagnose epilepsy; verify problems with loss of consciousness or dementia; verify brain activity for a person in a coma; study sleep disorders, monitor brain activity during surgery, and additional physical problems.

Multiple electrodes (typically 17-21, however there are standard positions for at least 70) are attached to a person's head during an EEG. The electrodes are referenced by the position of the electrode in relation to a lobe or area of a person's brain. The references are as follows: F = frontal; Fp = frontopolar; T = temporal; C = central; P = parietal; O = occipital; and A = auricular (ear electrode). Numerals are used to further narrow the position and "z" points relate to electrode sites in the midline of a person's head. An electrocardiogram ("EKG") may also appear on an EEG display.

The The EEG records brain waves from different amplifiers using various combinations of electrodes called montages. Montages are generally created to provide a clear picture of the spatial distribution of the EEG across the cortex. A montage is an electrical map obtained from a spatial array of recording electrodes and preferably refers to a particular combination of electrodes examined at a particular point in time.

In a bipolar montage, consecutive pairs of electrodes are linked by connecting the electrode input 2 of one channel to input 1 of the subsequent channel, so that adjacent channels have one electrode in common. The bipolar chains of electrodes may be connected going from front to back (longitudinal) or from left to right (transverse). In a bipolar montage signals between two active electrode sites are compared resulting in the difference in activity recorded. Another type of montage is the referential montage or monopolar montage. In a referential montage, various electrodes are connected to input 1 of each amplifier and a reference electrode is connected to input 2 of each amplifier. In a reference montage, signals are collected at an active electrode site and compared to a common reference electrode.

Reference montages are good for determining the true amplitude and morphology of a waveform. For temporal electrodes, CZ is usually a good scalp reference.

Being able to locate the origin of electrical activity ("localization") is critical to being able to analyze the EEG. Localization of normal or abnormal brain waves in bipolar montages is usually accomplished by identifying "phase reversal," a deflection of the two channels within a chain pointing to opposite directions. In a referential montage, all channels may show deflections in the same direction. If the electrical activity at the active electrodes is positive when compared to the activity at the reference electrode, the deflection will be downward. Electrodes where the electrical activity is the same as at the reference electrode will not show any deflection. In general, the electrode with the largest upward deflection represents the maximum negative activity in a referential montage.

Some patterns indicate a tendency toward seizures in a person. A physician may refer to these waves as "epileptiform abnormalities" or "epilepsy waves." These include spikes, sharp waves, and spike-and-wave discharges. Spikes and sharp waves in a specific area of the brain, such as the left temporal lobe, indicate that partial seizures might possibly come from that area. Primary generalized epilepsy, on the other hand, is suggested by spike-and-wave discharges that are widely spread over both hemispheres of the brain, especially if they begin in both hemispheres at the same time.

There are several types of brain waves: alpha waves, beta waves, delta wave, theta waves and gamma waves. Alpha waves have a frequency of 8 to 12 Hertz ("Hz"). Alpha waves are normally found when a person is relaxed or in a waking state when a person's eyes are closed but the person is mentally alert. Alpha waves cease when a person's eyes are open or the person is concentrating. Beta waves have a frequency of 13Hz to 30Hz. Beta waves are normally found when a person is alert, thinking, agitated, or has taken high doses of certain medicines. Delta waves have a frequency of less than 3Hz. Delta waves are normally found only when a person is asleep (non-REM or dreamless sleep) or the person is a young child. Theta waves have a frequency of 4Hz to 7Hz. Theta waves are normally found only when the person is asleep (dream or REM sleep) or the person is a young child. Gamma waves have a frequency of 30Hz to 100Hz. Gamma waves are normally found during higher mental activity and motor functions.

The following definitions are used herein.

"Amplitude" refers to the vertical distance measured from the trough to the maximal peak (negative or positive). It expresses information about the size of the neuron population and its activation synchrony during the component generation.

The term "analogue to digital conversion" refers to when an analogue signal is converted into a digital signal which can then be stored in a computer for further processing. Analogue signals are "real world" signals (e.g., physiological signals such as electroencephalogram, electrocardiogram or electrooculogram). In order for them to be stored and manipulated by a computer, these signals must be converted into a discrete digital form the computer can understand.

"Artifacts" are electrical signals detected along the scalp by an EEG, but that originate from non-cerebral origin. There are patient related artifacts (e.g., movement, sweating, ECG, eye movements) and technical artifacts (50/60 Hz artifact, cable movements, electrode paste-related).

The term "differential amplifier" refers to the key to electrophysiological equipment. It magnifies the difference between two inputs (one amplifier per pair of electrodes).

"Duration" is the time interval from the beginning of the voltage change to its return to the baseline. It is also a measurement of the synchronous activation of neurons involved in the component generation.

"Electrode" refers to a conductor used to establish electrical contact with a nonmetallic part of a circuit. EEG electrodes are small metal discs usually made of stainless steel, tin, gold or silver covered with a silver chloride coating. They are placed on the scalp in special positions.

"Electrode gel" acts as a malleable extension of the electrode, so that the movement of the electrodes leads is less likely to produce artifacts. The gel maximizes skin contact and allows for a low-resistance recording through the skin.

The term "electrode positioning" (10/20 system) refers to the standardized placement of scalp electrodes for a classical EEG recording. The essence of this system is the distance in percentages of the 10/20 range between Nasion-Inion and fixed points. These points are marked as the Frontal pole (Fp), Central (C), Parietal (P), occipital (O), and Temporal (T). The midline electrodes are marked with a subscript z, which stands for zero. The odd numbers are used as subscript for points over the left hemisphere, and even numbers over the right

"Electroencephalogram" or "EEG" refers to the tracing of brain waves, by recording the electrical activity of the brain from the scalp, made by an electroencephalograph.

"Electroencephalograph" refers to an apparatus for detecting and recording brain waves (also called *encephalograph).*

"Epileptiform" refers to resembling that of epilepsy.

"Filtering" refers to a process that removes unwanted frequencies from a signal.

"Filters" are devices that alter the frequency composition of the signal.

"Montage" means the placement of the electrodes. The EEG can be monitored with either a bipolar montage or a referential one. Bipolar means that there are two electrodes per one channel, so there is a reference electrode for each channel. The referential montage means that there is a common reference electrode for all the channels.

"Morphology" refers to the shape of the waveform. The shape of a wave or an EEG pattern is determined by the frequencies that combine to make up the waveform and by their phase and voltage relationships. Wave patterns can be described as being: "Monomorphic". Distinct EEG activity appearing to be composed of one dominant activity. "Polymorphic", distinct EEG activity composed of multiple frequencies that combine to form a complex waveform. "Sinusoidal". Waves resembling sine waves. Monomorphic activity usually is sinusoidal. "Transient". An isolated wave or pattern that is distinctly different from background activity.

"Spike" refers to a transient with a pointed peak and a duration from 20 to under 70 msec.

The term "sharp wave" refers to a transient with a pointed peak and duration of 70-200 msec.

The term "neural network algorithms" refers to algorithms that identify sharp transients that have a high probability of being epileptiform abnormalities.

"Noise" refers to any unwanted signal that modifies the desired signal. It can have multiple sources.

"Periodicity" refers to the distribution of patterns or elements in time (e.g., the appearance of a particular EEG activity at more or less regular intervals). The activity may be generalized, focal or lateralized.

An EEG epoch is an amplitude of a EEG signal as a function of time and frequency.

Various techniques have been developed to present the EEG data to a physician or technician, see e.g. WO2008/057365 A2. However, these techniques are still lacking. If the raw EEG report is presented to a physician or technician, then artifacts typically render the EEG report incapable of distinguishing brain activity such as a seizure from artifacts. Despite the use of artifact reduction algorithms, the failure to accurately distinguish true physiological rhythmicity from the artifacts is a serious shortcoming of current software systems and requires an expert assessment.

An EEG report produces tremendous amounts of information about a person's brain activity. However, there is a need to quickly and easily interpret that information in order to properly analyze the brain activity of a person.

### Summary of the Invention

The invention is defined by the subject-matter of the independent claims. Preferred embodiments are defined in the dependent claims.

The present invention is a system for analyzing an EEG recording as defined in appended claim 1. The system includes a plurality of electrodes, at least one amplifier, a processor and a display. The plurality of electrodes generates a plurality of EEG signals. The at least one amplifier is connected to each of the plurality of electrodes by a plurality of wires to amplify each of the plurality of EEG signals. The processor is connected to the amplifier to generate an EEG recording from the plurality of EEG signals. The display is connected to the processor for displaying an EEG recording. The processor is configured to organize a plurality of detections of the processed EEG recording by spike focus.

In a preferred embodiment of the invention, the processor is configured to create a back-to-back view of spike detections organized by spike focus.

In a preferred embodiment of the invention, the processor is configured to select an EEG clip of a spike focus of the processed EEG recording to view an extended portion of the EEG for context.

According to the present invention, the processor is configured to average a plurality of detections of the processed EEG recording by spike focus on a summary.

The processor may be configured to move from an average of a plurality of detections of the processed EEG recording by spike focus to an individual detection.

The processor may be configured to mark a plurality of spike averages of the processed EEG recording and a plurality of individual detections of the processed EEG recording at spike focus.

The processor may be configured to determine which of a plurality of detections of the processed EEG recording to include in a final analysis by changing a sensitivity of the EEG to view a detection.

In a preferred embodiment, the processor is configured to process the EEG recording with a plurality of neural network algorithms to create a processed EEG recording.

### Brief Description of the Drawings

FIG. 1 is a block diagram of a system for analyzing an EEG recording.
FIG. 2 is an illustration of an analyzed EEG recording.
FIG. 3 is an illustration of a raw detection display of an analyzed EEG recording.
FIG. 4 is an illustration of an expanded detection view display of an analyzed EEG recording.
FIG. 5 is an illustration of a final report display of an analyzed EEG recording.
FIG. 6 is a flow chart of a general method for analyzing an EEG recording.
FIG. 7 is an illustration of an EEG recording for a normal awake patient.
FIG. 7A is an illustration of an analyzed EEG recording for a generalized spike EEG.
FIG. 7B is an illustration of an analyzed EEG recording for a focal spike EEG.
FIG. 8 is a block diagram of a system for analyzing an EEG recording.
FIG. 9 is a flow chart of a general method for analyzing an EEG recording.
FIG. 10 is a flow chart of a specific method for analyzing an EEG recording.

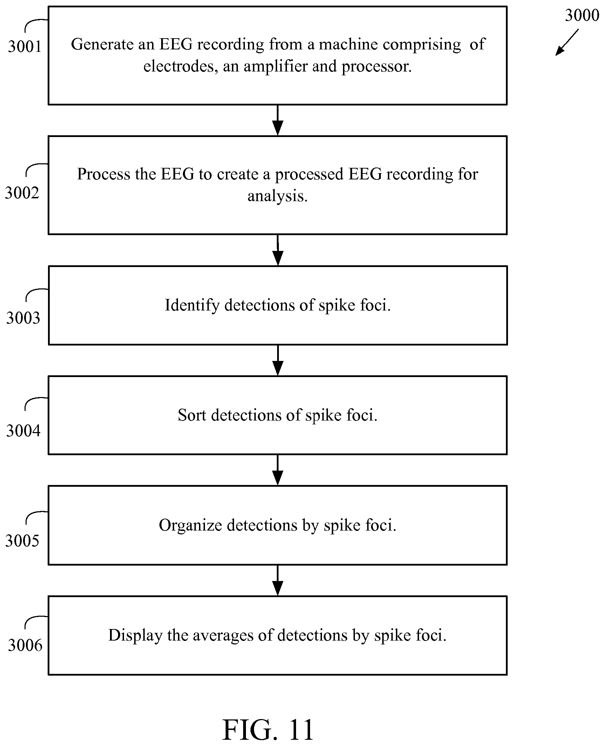

FIG. 11 is a flow chart of a specific method for analyzing an EEG recording.
FIG. 12 is a map representing the international 10-20 electrode system for electrode placement for an EEG.
FIG. 13 is a detailed map representing the intermediate 10% electrode positions, as standardized by the American Electroencephalographic Society, for electrode placement for an EEG.

### Best Mode(s) For Carrying Out The Invention

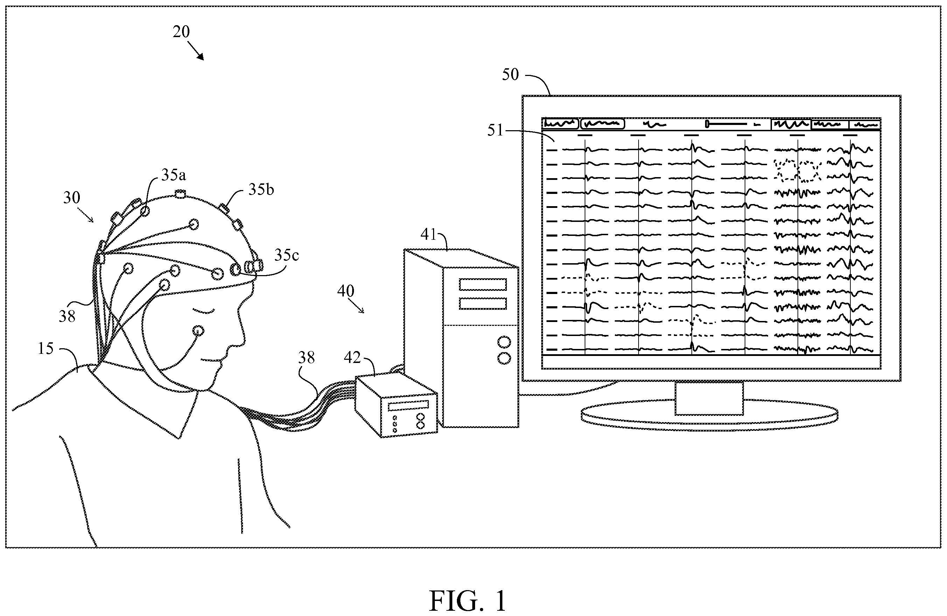

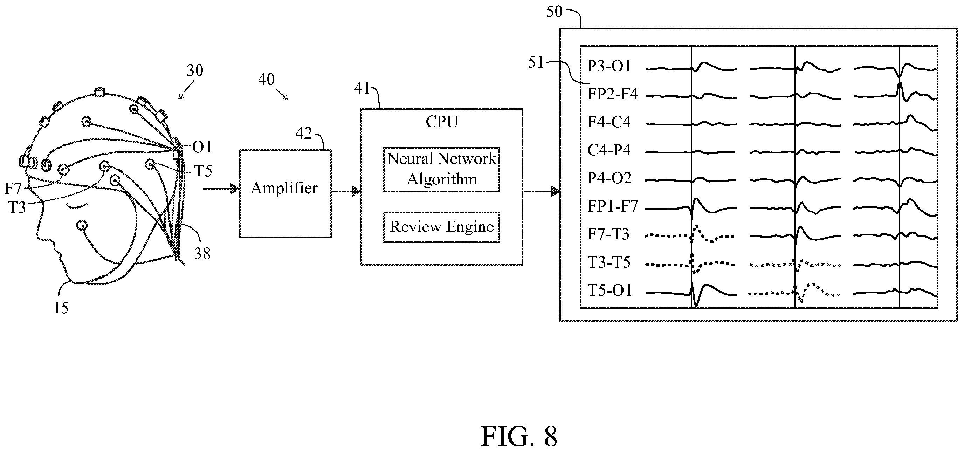

As shown in FIG. 1, an EEG system is generally designated 20. The system preferably includes a patient component 30, an EEG machine component 40 and a display component 50. The patient component 30 includes a plurality of electrodes 35a, 35b, 35c attached to the patient 15 and wired by cables 38 to the EEG machine component 40. The EEG machine component 40 preferably comprises a CPU 41 and an amplifier component 42. The EEG machine component 40 is connected to the display component 50 for display of the combined EEG reports, and for switching from a processed EEG report to the combined EEG reports, or from the processed EEG report to an original EEG report. As shown in FIG. 8, the EEG machine component 40 preferably includes a review engine and neural network algorithms. The machine component also preferably comprises a memory, a memory controller, a microprocessor, a DRAM, and an Input/Output. Those skilled in the pertinent art will recognize that the machine component 40 may include other components without departing from the scope of the present invention. The EEG recordings are then processed using neural network algorithms to generate a processed EEG recording which is analyzed for display.

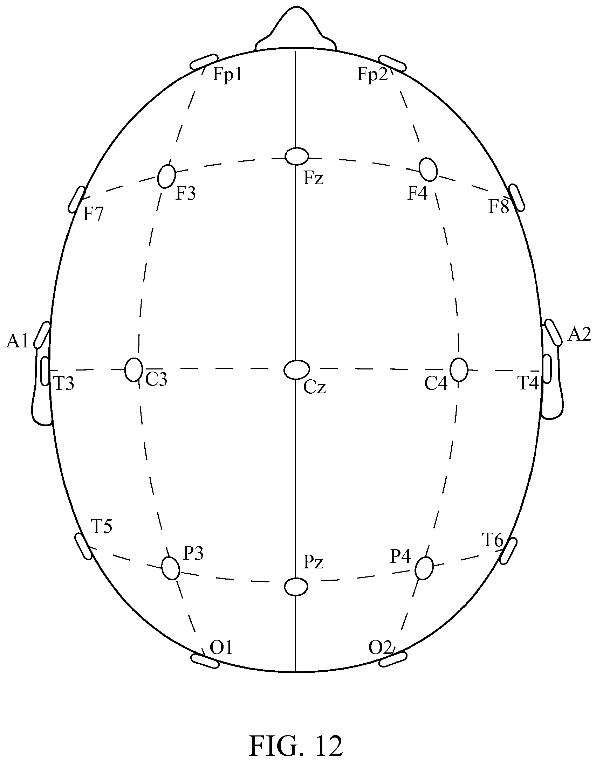

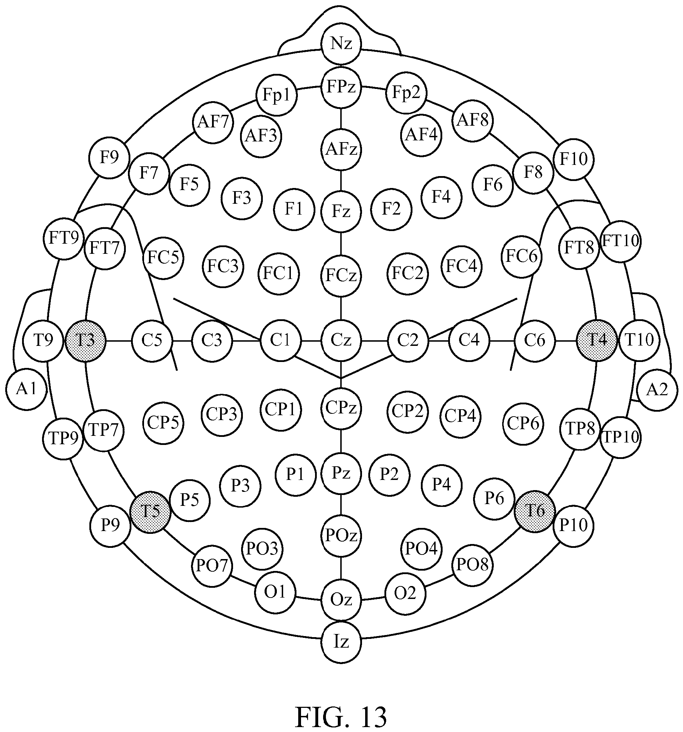

A patient has a plurality of electrodes attached to the patient's head with wires from the electrodes connected to an amplifier for amplifying the signal to a processor which is used to analyze the signals from the electrodes and create an EEG recording. The brain produces different signals at different points on a patient's head. Multiple electrodes are positioned on a patient's head as shown in FIGS. 12 and 13. For example Fp1 on FIG. 12 is represented in channel FP1-F7 on FIG. 8. The number of electrodes determines the number of channels for an EEG. A greater number of channels produces a more detailed representation of a patient's brain activity. Preferably, each amplifier of an EEG machine component 40 corresponds to two electrodes attached to a patient's head. The output from an EEG machine component is the difference in electrical activity detected by the two electrodes. The placement of each electrode is critical for an EEG report since the closer electrode pairs are to each other, the less difference in the brainwaves that are recorded by the EEG machine component. A more thorough description of an electrode utilized with the present invention is detailed in Wilson et al, U.S. Patent Number 8112141 for a *Method And Device For Quick Press On EEG Electrode.* The EEG is optimized for automated artifact filtering. The EEG recordings are then processed using neural network algorithms to generate a processed EEG recording which is analyzed for display.

Algorithms for removing artifacts from EEG typically use Blind Source Separation (BSS) algorithms like CCA (canonical correlation analysis) and ICA (Independent Component Analysis) to transform the signals from a set of channels into a set of component waves or "sources." The sources that are judged as containing artifact are removed and the rest of the sources are reassembled into the channel set.

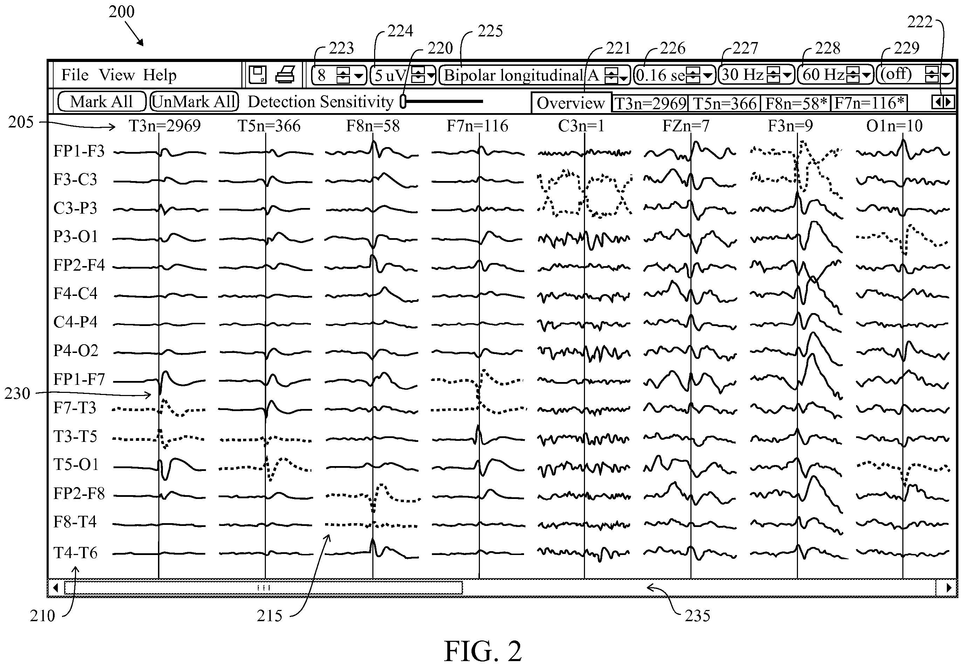

FIGS. 2-5 illustrate analyzed EEG recordings. As shown in FIG. 2, a display of an analyzed EEG recording on a computer screen is designated 200. Reference 205 designates the electrode foci (T3) and the number of detections (2969) selected at this sensitivity. The montage bar is designated 210 and allows for montage controls. Reference 215 shows a primary electrode detection focus. The detection sensitivity slider is designated 220 and allows an operator to select the sensitivity for display. Dragging the slider to the right dynamically increases the detection sensitivity thereby yielding more true positives but also more false positives. Less sensitivity shows less spikes. The group tab is designated 221, and the tab is used to select the detection group displayed in the main window. The following types of tabs are available: Overview which is detection averages arranged by electrode focus, showing averages of all detections at chosen detection sensitivity; Individual Electrode Foci, for example T3, T5; Final Report which is spike averages of hand chosen detections, sorted by electrode focus. The number of detections is shown at each focus for the chosen sensitivity. The navigation tabs are designated 222, which allow for navigation to other tabs not currently in view on the window. The spike detections per page tab is designated 223 and allows for a number of detections that yields about 30 mm per each one second spike detection event. The EEG voltage amplitude selector is designated 224. The montage selector tab is 225, the LFF tab is 226, the HFF tab is 227, the notch tab is 228, and the customer filter tab is 229. An operator can jump to a group's constituent spikes by clicking on the group such as at point 230. The page forward tab is 235.

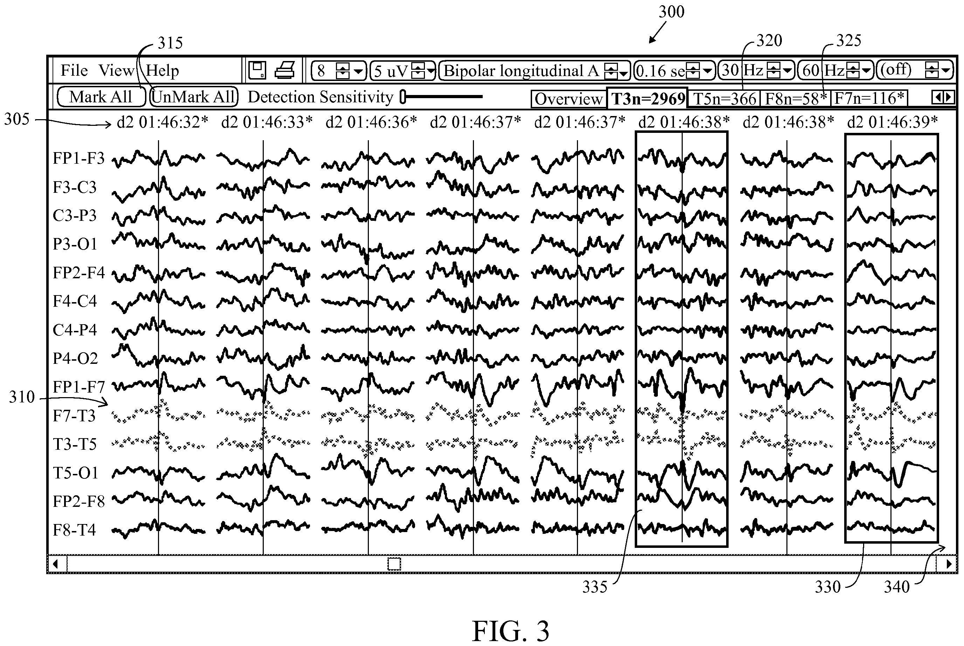

As shown in FIG. 3, a display showing raw detections at T3 of an analyzed EEG recording on a computer screen is designated 300. A time of detection is designated 305. Electrodes involved in the detection are typically highlighted, and shown as reference 310. The mark or unmark tab is 315, which allows for marked detections to appear in the final report. The navigate tab 320 allows for navigation between detection foci. As shown at 325, detections that have already been viewed are marked with an asterisk. A hand marked detection 330 places a box around the detection. EEG centered on the spike detection is shown at 335. Tab 340 allows for movement to the next page of detections.

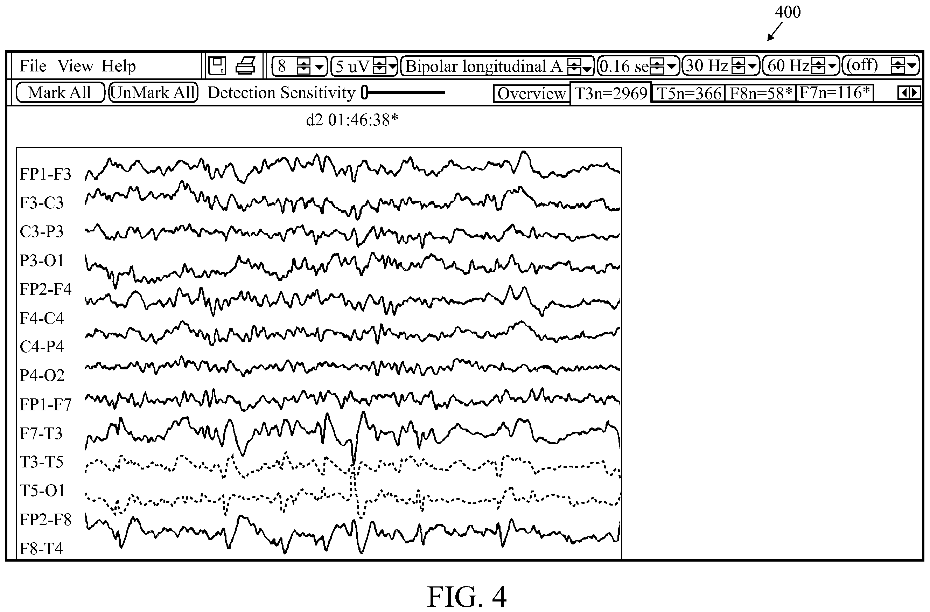

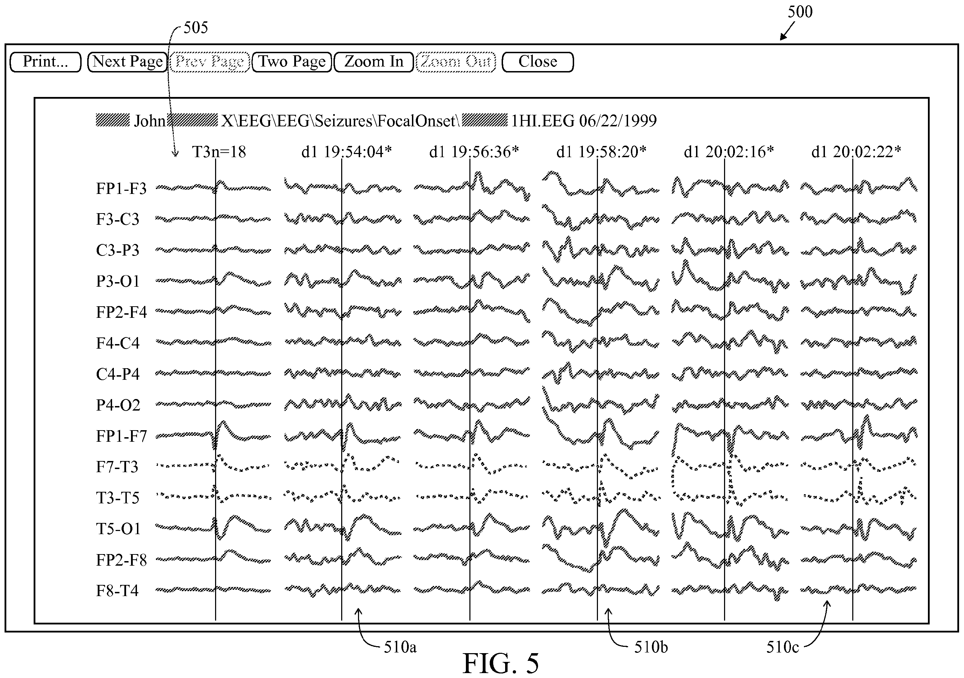

As shown in FIG. 4, a display 400 of an analyzed EEG recording on a computer screen shows an expanded detection view. As shown in FIG. 5, a final report display of an analyzed EEG recording on a computer screen is designated 500. An average of user selected spikes with T3 voltage maximum is shown at reference 505. 510a, 510b and 510c are individual constituent user selected spikes.

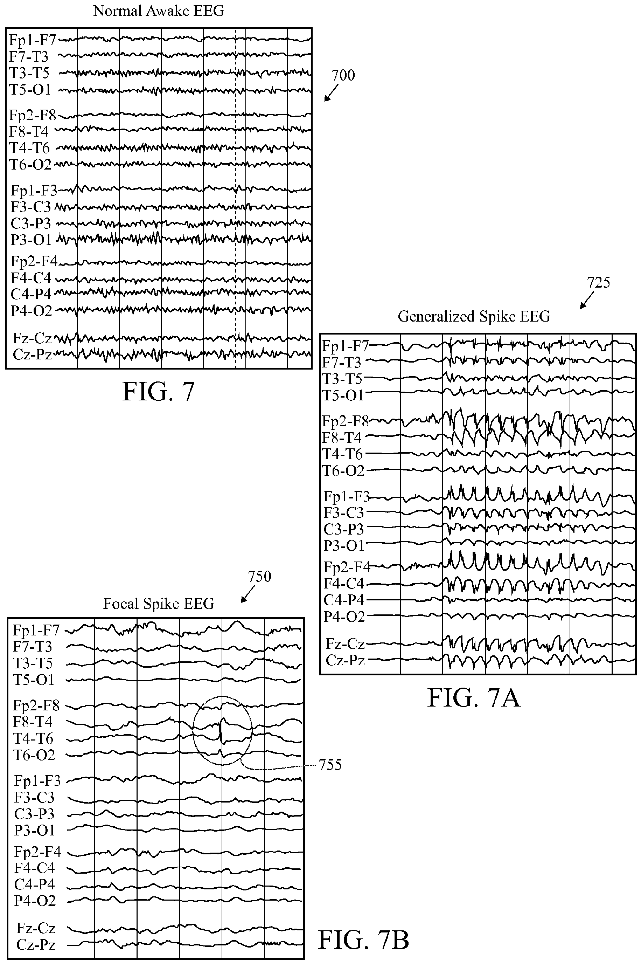

An EEG 700 for a normal awake patient is shown in FIG. 7. An EEG 725 having generalized spikes is shown in FIG. 7A. An EEG 750 having a focal spike is shown in FIG. 7B.

In use for analyzing an EEG recording, a technician or physician activates the review program, and after a few seconds the review program opens. The overview window is initially presented. The overview depicts averages from the various spike foci detected by a spike detection mechanism. To create these overview averages the spike detections are sorted by detection foci (electrode) and then all detections at a particular focus are mathematically averaged. For example, the first column of EEG represents an average of 2969 events that had their maximum point of detection at the T3 electrode. The columns of the EEG are preferably separated from other columns by a thin band of white. Each EEG column represents a distinct group average. The primary electrode focal point of each average, and the number of detection events incorporated into each average, are shown above the columns of EEG. Channels including the detection focal point electrode are highlighted red. As with evoked potentials, averaging multiple detections results in an increase in the signal-to-noise ratio and makes it easier to delineate the field of distribution of epileptiform abnormalities.

The sensitivity of the SpikeDetector output can be dynamically adjusted during the review process. This is done by using the detection sensitivity slider, which is labeled. When Easy SpikeReview is initially opened, the detection sensitivity slider is set to the far left position. In this position the SpikeDetector neural network algorithms identify sharp transients that have a high probability of being epileptiform abnormalities: these are events the detector assigned a high probability of being a real epileptiform abnormality. The rate of false positive detections at this setting is lowest. Thus, the ratio of true epileptiform signal to false positive noise is highest at this setting. However, some spikes and sharp waves that are less well-formed may not be evident with the slider set at its lowest sensitivity. The detector's sensitivity can be quickly adjusted by dragging the slider towards the right so that it is more sensitive and thus more likely to identify less well-formed or lower amplitude transients. New groups may then appear in the overview display of spike averages. In concert with the increase in true spike detections, there is also an increase in false positive detections.

An example of changes in the number of detected events associated with moving the detection sensitivity adjustment slider from the left to the far right (see red arrows) is shown. Looking only at the T3 detections, the number of detected events increased from 2969 to 4528 (see yellow highlights).

In records with rare epileptiform abnormalities or those in which the SpikeDetector neural networks, when set to lowest sensitivity, do not recognize the epileptiform abnormalities well, switching to the highest setting on the detection sensitivity slider may allow visualization of real epileptiform abnormalities. In such cases, identifying the rare events often requires assessment of the individual raw detections. This is accomplished by either displaying all raw detections back-to-back following the spike averages on the overview page, or by reviewing the detections at each electrode location by progressively selecting the location tabs at the top of the EEG window (see below).

Clicking on any of the electrode location tabs at the top of the EEG window will display the raw (non-averaged) spike detections that arose from that particular electrode location. The individual detections are separated by a thin band of white, and the detection point is centered in a one second segment of EEG and indicated by a faint vertical gray line. Left double-clicking with the mouse on any individual detection will cause an expanded EEG view of that particular detection to appear. Left double-clicking on the expanded view will return the user to a display of back-to-back individual detections.

When viewing individual spike detections (accessed from the tabs above the EEG window), exemplar spikes can be hand-marked by left-clicking with the mouse on the desired example. A rectangle outlining the chosen spike will appear. Hand-marked detections will be included in the spike averages that appear in the FinalReport. These hand-marked events can also be displayed back-to-back, immediately following their averages in FinalReport, and can be printed for archival purposes or for evaluation by another reviewer.

Clicking on FinalReport at the top of the EEG window displays a summary of all hand-marked events. The initial default view shows the mathematical averages of the user-chosen hand-marked events, sorted by electrode focus. As explained, head voltage topograms and back-to-back individual user-selected events are displayed by selecting menu options or via right mouse click choices. Voltage topograms are only created when viewing the EEG in a referential montage.

Using the initial overview screen with the detection sensitivity slider set to the far left (lowest sensitivity), identify any clear-cut or probable epileptiform abnormalities and note their locations. Double-click on the epileptiform abnormalities to further verify their nature by viewing individual examples contributing to the averages. Mark exemplar individual events as desired (for a Final Report).

Slide the detection sensitivity slider to the far right (highest sensitivity) and reassess the overview screen to determine whether any other clear-cut or probable epileptiform abnormalities become evident.

If epileptiform abnormalities were discovered and verified via the overview display, proceed with further review of back-to-back raw detections, as indicated.

If no epileptiform abnormalities were evident on overview, methodically assess all raw detections by selecting the various location tabs and paging through the back-to-back event detections. If any real epileptiform abnormalities are discovered, click on these to mark them for inclusion in the Final Report and then continue a review.

Once a review is completed, go to Final Report. On the Final Report, choose whether to display only averages of epileptiform abnormalities or both averages and back-to-back hand-selected events. Print the Final Report examples, if desired.

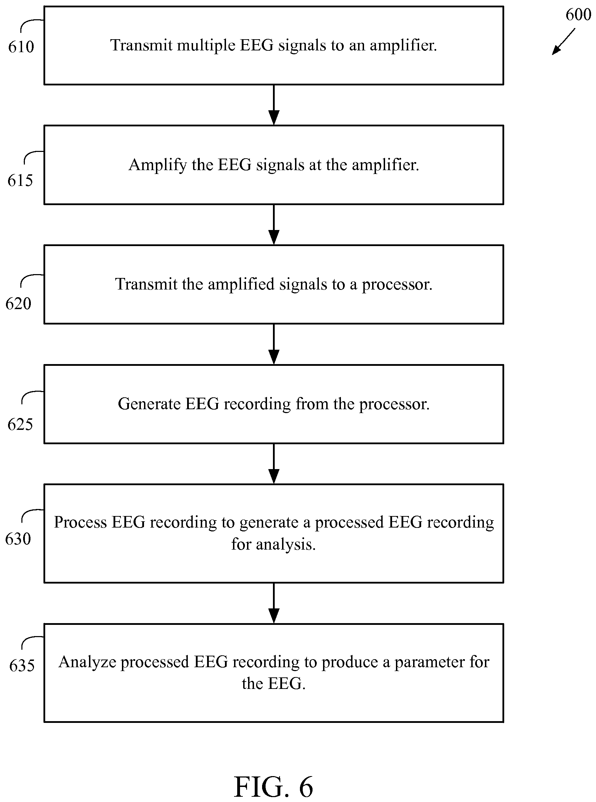

As shown in FIG. 6, a general method for analyzing an EEG recording is designated 600. At block 610, multiple EEG signals are transmitted to an amplifier. At block 615, the EEG signals are amplified by the amplifier. At block 620, the amplified signals are transmitted to a processor. At block 625, an EEG recording is generated by the processor. At block 630, the EEG recording is processed to generate a processed EEG recording for analysis. Processing preferably involves performing artifact reduction on the raw EEG recording. At block 635, the processed EEG recording is analyzed to produce a parameter for the EEG.

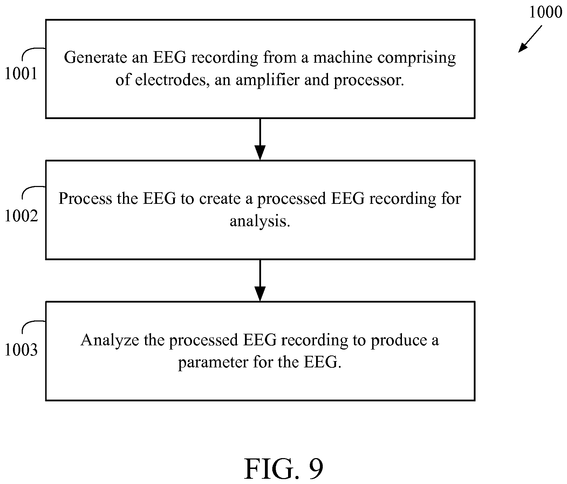

As shown in FIG. 9, a general method for analyzing an EEG recording is designated 1000. At block 1001, an EEG recording is generated from a machine comprising electrodes, an amplifier and a processor. At block 1002, the EEG recording is processed to create a processed EEG recording for analysis. At block 1003, the processed EEG recording is analyzed to produce a parameter for the EEG.

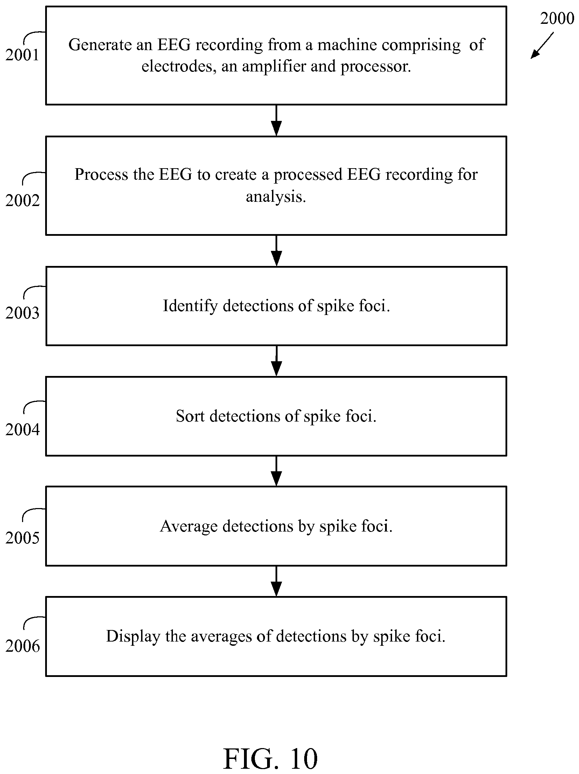

As shown in FIG. 10, a specific method for analyzing an EEG recording is designated 2000. At block 2001, an EEG recording is generated from a machine comprising electrodes, an amplifier and a processor. At block 2002, the EEG recording is processed to create a processed EEG recording for analysis. At block 2003, the detections by spike foci are identified in the processed EEG recording. At block 2004, the detections by spike foci are sorted, preferably by electrodes. At block 2005, the detections of spike foci are averaged. At block 2006, the averages of detections by spike foci are displayed for a physician or technician.

As shown in FIG. 1 1, a specific method for analyzing an EEG recording is designated 3000. At block 3001, an EEG recording is generated from a machine comprising electrodes, an amplifier and a processor. At block 3002, the EEG recording is processed to create a processed EEG recording for analysis. At block 3003, the detections by spike foci are identified in the processed EEG recording. At block 3004, the detections by spike foci are sorted, preferably by electrodes. At block 3005, the detections of spike foci are organized. At block 3006, the organization of detections by spike foci are displayed for a physician or technician.

## Claims

1. A system for analyzing an electroencephalogram (EEG) recording, the system comprising:
a plurality of electrodes (35a, 35b, 35c) for generating a plurality of EEG signals;
at least one amplifier (42) connected to each of the plurality of electrodes by a plurality of wires to amplify each of the plurality of EEG signals;
a processor (41) connected to the amplifier to generate an EEG recording from the plurality of EEG signals, the EEG recording comprising a plurality of channels displaying brain activity, each channel of the plurality of channels corresponding to a difference in the electrical activity detected by two electrodes of the plurality of electrodes; and
a display (50) connected to the processor for displaying an EEG recording;
wherein the processor is configured to process the EEG recording to create a processed EEG recording for analysis;
wherein spike detections by spike foci are identified in the processed EEG recording, wherein each spike of the spike foci is an isolated wave or pattern distinct from a background activity of the processed EEG recording, and each isolated wave or pattern has a pointed peak and a duration ranging from 20 to 70 milliseconds;
wherein the processor is configured to sort the spike detections by spike foci in the processed EEG recording according to each of the plurality of electrodes; and
and to display the averages of the spike detections by spike foci on the display of the system;
wherein a spike detection sensitivity slider (220) allows selection of sensitivity for display, such that detections corresponding to sharp transients that have a high probability of being epileptiform abnormalities are displayed when the spike detection sensitivity slider is in a first position and detections corresponding to both the sharp transients that have a high probability of being epileptiform abnormalities and lower amplitude transients are displayed when the spike detection sensitivity slider is in a second position, wherein the second position corresponds to a higher sensitivity than the first position.

2. The system according to claim 1 wherein the processor is further configured to create a back-to-back view of spike detections organized by spike focus.

3. The system according to claim 1 wherein the processor is further configured to select an EEG clip of a spike focus to view an extended portion of the EEG for context.

4. The system according to claim 1 wherein the processor is configured to process the EEG recording with a plurality of neural network algorithms.

## Patentansprüche

1. System zum Analysieren einer Elektroenzephalogramm-Aufnahme (EEG-Aufnahme), wobei das System umfasst:
eine Vielzahl von Elektroden (35a, 35b, 35c) zum Generieren einer Vielzahl von EEG-Signalen;
zumindest einen Verstärker (42), der mit jeder der Vielzahl von Elektroden durch eine Vielzahl von Drähten verbunden ist, um jedes der Vielzahl von EEG-Signalen zu verstärken;
einen Prozessor (41), der mit dem Verstärker verbunden ist, um eine EEG-Aufnahme aus der Vielzahl von EEG-Signalen zu generieren, wobei die EEG-Aufnahme eine Vielzahl von Kanälen umfasst, die Gehirnaktivität anzeigen, wobei jeder Kanal der Vielzahl von Kanälen einer Differenz in der elektrischen Aktivität entspricht, die durch zwei Elektroden der Vielzahl von Elektroden detektiert wird; und
eine Anzeige (50), die mit dem Prozessor verbunden ist, um eine EEG-Aufnahme anzuzeigen;
wobei der Prozessor konfiguriert ist, um die EEG-Aufnahme zu verarbeiten, um eine verarbeitete EEG-Aufnahme zur Analyse zu erzeugen;
wobei Spike-Detektionen durch Spike-Foci in der verarbeiteten EEG-Aufnahme identifiziert werden, wobei jeder Spike der Spike-Foci eine isolierte Welle oder ein isoliertes Muster ist, das sich von einer Hintergrundaktivität der verarbeiteten EEG-Aufnahme unterscheidet, und jede isolierte Welle oder jedes isolierte Muster eine pointierte Spitze und eine Dauer von 20 bis 70 Millisekunden aufweist;
wobei der Prozessor konfiguriert ist, um die Spike-Detektionen durch Spike-Foci in der verarbeiteten EEG-Aufnahme gemäß jeder der Vielzahl von Elektroden zu sortieren; und
um die Mittelwerte der Spike-Detektionen durch Spike-Foci auf der Anzeige des Systems anzuzeigen;
wobei ein Empfindlichkeitsschieber (220) für die Spike-Detektion eine Auswahl einer Empfindlichkeit zur Anzeige ermöglicht, so dass Detektionen, die scharfen Übergängen entsprechen, die eine hohe Wahrscheinlichkeit haben epileptiforme Anomalien zu sein, angezeigt werden, wenn sich der Empfindlichkeitsschieber für die Spike-Detektion in einer ersten Position befindet, und sowohl Detektionen, die den scharfen Übergängen entsprechen, die eine hohe Wahrscheinlichkeit haben epileptiforme Anomalien zu sein, als auch Detektionen, die Übergängen mit niedrigerer Amplitude entsprechen, angezeigt werden, wenn sich der Empfindlichkeitsschieber für die Spike-Detektion in einer zweiten Position befindet, wobei die zweite Position einer höheren Empfindlichkeit entspricht als die erste Position.

2. System nach Anspruch 1, wobei der Prozessor ferner konfiguriert ist, um eine Rücken-zu-Rücken-Ansicht von Spike-Detektionen zu erzeugen, die nach Spike-Fokus organisiert sind.

3. System nach Anspruch 1, wobei der Prozessor ferner konfiguriert ist, um einen EEG-Clip eines Spike-Fokus auszuwählen, um einen erweiterten Teil des EEGs im Kontext zu betrachten.

4. System nach Anspruch 1, wobei der Prozessor konfiguriert ist, um die EEG-Aufnahme mit einer Vielzahl von neuronalen Netzwerkalgorithmen zu verarbeiten.

## Revendications

1. Système pour analyser un enregistrement d'électroencéphalogramme (EEG), le système comprenant :
une pluralité d'électrodes (35a, 35b, 35c) pour générer une pluralité de signaux EEG ;
au moins un amplificateur (42) connecté à chacune de la pluralité d'électrodes par une pluralité de fils pour amplifier chacun de la pluralité de signaux EEG ;
un processeur (41) connecté à l'amplificateur pour générer un enregistrement EEG à partir de la pluralité de signaux EEG, l'enregistrement EEG comprenant une pluralité de canaux affichant une activité cérébrale, chaque canal de la pluralité de canaux correspondant à une différence dans l'activité électrique détectée par deux électrodes de la pluralité d'électrodes ; et
un affichage (50) connecté au processeur pour afficher un enregistrement EEG ;
dans lequel le processeur est configuré pour traiter l'enregistrement EEG afin de créer un enregistrement EEG traité pour analyse ;
dans lequel des détections de pointe par des foyers de pointe sont identifiées dans l'enregistrement EEG traité, dans lequel chaque pointe des foyers de pointe est une onde ou un motif isolé(e) distinct(e) d'une activité de fond de l'enregistrement EEG traité, et chaque onde ou motif isolé(e) a un pic pointu et une durée allant de 20 à 70 millisecondes ;
dans lequel le processeur est configuré pour trier les détections de pointe par des foyers de pointes dans l'enregistrement EEG traité selon chacune de la pluralité d'électrodes ; et
pour afficher les moyennes des détections de pointe par foyers de pointe sur l'affichage du système ;
dans lequel un curseur de sensibilité de détection de pointe (220) permet une sélection de sensibilité pour un affichage, de telle sorte que des détections correspondant à des transitoires aigus qui ont une forte probabilité d'être des anomalies épileptidiques sont affichées lorsque le curseur de sensibilité de détection de pointe est dans une première position et des détections correspondant à la fois aux transitoires aigus qui ont une forte probabilité d'être des anomalies épileptidiques et à des transitoires d'amplitude inférieure sont affichées lorsque le curseur de sensibilité de détection de pointe est dans une seconde position, dans lequel la seconde position correspond à une sensibilité supérieure à la première position.

2. Système selon la revendication 1, dans lequel le processeur est en outre configuré pour créer une vue dos à dos de détections de pointe organisées par foyer de pointe.

3. Système selon la revendication 1, dans lequel le processeur est en outre configuré pour sélectionner un clip EEG d'un foyer de pointe pour visualiser une partie étendue de l'EEG pour le contexte.

4. Système selon la revendication 1, dans lequel le processeur est configuré pour traiter l'enregistrement EEG avec une pluralité d'algorithmes de réseau neuronal.
